# EUROPEAN PATENT APPLICATION

(11) **EP 4 541 927 A2**
(43) Date of publication of application: **23.04.2025**
(21) Application number: 24206256.0
(22) Date of filing: 11.10.2024
(51) Int. Cl.: C23C 14/16, C23C 14/18

(54) **COATED ARTICLE WITH TFMG SUPPORT LAYER**

(30) Priority: 16.10.2023 US 202363544254 P
(71) Applicant: Vapor Technologies, Inc., Longmont CO 80503 (US)
(72) Inventor: SULLIVAN, Patrick Anthony, Longmont, 80504 (US); ANTON, Bryce Randolph, Longmont, 80503 (US)
(74) Representative: Keltie LLP

(57) **Abstract**

A coated article includes a doped TFMG layer (16) and/or undoped TFMG layer (14) disposed over and optionally contacting a substrate (12). A top layer (20) is disposed over and optionally contacts the TFMG layer (16). The top layer (20) includes a metal-containing layer and/or a carbon-containing layer. The coated article can also include a doped TFMG layer (16) and an undoped TFMG layer (14) with a leveling primer layer (24).

## Description

### TECHNICAL FIELD

In at least one aspect, the present invention is related to coated articles that include one or more thin film metallic glass layers.

### BACKGROUND

Electroplating is a coating process used to provide corrosion-resistant and decorative metal or metal alloy coatings to a variety of objects. Although electroplating works well, it presents a number of environmental concerns. Therefore, metallic or metal compound PVD layers have been used to replace the Ni in the conventional Ni-Cr electroplating stacks used for plumbing, door hardware, and automotive trim applications. Due to the columnar microstructure of the thin film process, the corrosion resistance and durability of PVD layers (e.g. pure Ni) are usually inferior to conventional Ni electroplating.

Accordingly, there is a need for improved environmentally friendly coating methods for applying corrosion-resistant and decorative metal coatings.

### SUMMARY

In at least one aspect, a coated article is provided. The coated article includes a substrate and a doped thin film metallic glass (TFMG) and/or undoped thin film metallic glass (TFMG) layer disposed over the substrate. A top layer is disposed over and optionally contacts the doped TFMG layer and/or the undoped TFMG layer. Advantageously, the top layer includes a metal-containing layer or carbon-containing layer.

In another aspect, the coated article includes an undoped TFMG, a doped TFMG layer and a leveling primer layer.

In another aspect, a method for making a coated article is provided. The method includes the steps of depositing a doped thin film metallic glass (TFMG) and/or undoped thin film metallic glass (TFMG) layer over a substrate and depositing a top layer by physical vapor deposition over the doped TFMG layer and/or the undoped TFMG layer. Advantageously, the top layer includes a metal-containing layer or carbon-containing layer.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

For a further understanding of the nature, objects, and advantages of the present disclosure, reference should be had to the following detailed description, read in conjunction with the following drawings, wherein like reference numerals denote like elements and wherein:
FIGURE 1. Schematic cross-section of a coated article that includes a TFMG layer and a top layer.
FIGURE 2. Schematic cross-section of a coated article that includes a TFMG layer, a doped TFMG layer, and a top layer.
FIGURE 3. Schematic cross-section of a coated article that includes a leveling primer layer, a TFMG layer, and a top layer.
FIGURE 4. Schematic cross-section of a coated article that includes a leveling primer layer, a TFMG layer, a doped TFMG layer, and a top layer.
FIGURE 5A, 5B, 5C, and 5D. Schematic flowcharts depicting methods of making the coated article.

### DETAILED DESCRIPTION

Reference will now be made in detail to presently preferred compositions, embodiments, and methods of the present invention, which constitute the best modes of practicing the invention presently known to the inventors. The Figures are not necessarily to scale. However, it is to be understood that the disclosed embodiments are merely exemplary of the invention that may be embodied in various and alternative forms. Therefore, specific details disclosed herein are not to be interpreted as limiting, but merely as a representative basis for any aspect of the invention and/or as a representative basis for teaching one skilled in the art to variously employ the present invention.

Except in the examples, or where otherwise expressly indicated, all numerical quantities in this description indicating amounts of material or conditions of reaction and/or use are to be understood as modified by the word "about" in describing the broadest scope of the invention. Practice within the numerical limits stated is generally preferred. Also, unless expressly stated to the contrary: percent, "parts of," and ratio values are by weight; the term "polymer" includes "oligomer," "copolymer," "terpolymer," and the like; molecular weights provided for any polymers refers to weight average molecular weight unless otherwise indicated; the description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

It is also to be understood that this invention is not limited to the specific embodiments and methods described below, as specific components and/or conditions may, of course, vary. Furthermore, the terminology used herein is used only for the purpose of describing particular embodiments of the present invention and is not intended to be limiting in any way.

It must also be noted that, as used in the specification and the appended claims, the singular form "a," "an," and "the" comprise plural referents unless the context clearly indicates otherwise. For example, reference to a component in the singular is intended to comprise a plurality of components.

The term "comprising" is synonymous with "including," "having," "containing," or "characterized by." These terms are inclusive and open-ended and do not exclude additional, unrecited elements or method steps.

The phrase "consisting of" excludes any element, step, or ingredient not specified in the claim. When this phrase appears in a clause of the body of a claim, rather than immediately following the preamble, it limits only the element set forth in that clause; other elements are not excluded from the claim as a whole.

The phrase "consisting essentially of' limits the scope of a claim to the specified materials or steps, plus those that do not materially affect the basic and novel characteristic(s) of the claimed subject matter.

With respect to the terms "comprising," "consisting of," and "consisting essentially of," where one of these three terms is used herein, the presently disclosed and claimed subject matter can include the use of either of the other two terms.

The phrase "composed of" means "including" or "comprising." Typically, this phrase is used to denote that an object is formed from a material.

It should also be appreciated that integer ranges explicitly include all intervening integers. For example, the integer range 1-10 explicitly includes 1, 2, 3, 4, 5, 6, 7, 8, 9, and 10. Similarly, the range 1 to 100 includes 1, 2, 3, 4.... 97, 98, 99, 100. Similarly, when any range is called for, intervening numbers that are increments of the difference between the upper limit and the lower limit divided by 10 can be taken as alternative upper or lower limits. For example, if the range is 1.1. to 2.1 the following numbers 1.2, 1.3, 1.4, 1.5, 1.6, 1.7, 1.8, 1.9, and 2.0 can be selected as lower or upper limits. In the specific examples set forth herein, concentrations, temperature, and reaction conditions (e.g. pressure, pH, etc.) can be practiced with plus or minus 50 percent of the values indicated rounded to three significant figures. In a refinement, concentrations, temperature, and reaction conditions (e.g., pressure, pH, etc.) can be practiced with plus or minus 30 percent of the values indicated rounded to three significant figures of the value provided in the examples. In another refinement, concentrations, temperature, and reaction conditions (e.g., pH, etc.) can be practiced with plus or minus 10 percent of the values indicated rounded to three significant figures of the value provided in the examples.

### Abbreviations:

"ABS" means acrylonitrile butadiene styrene.

"at.%" means atom percent.

"HiPIMS" means high-power impulse magnetron sputtering.

"PVD" means physical vapor deposition.

"TFMG" means thin film metallic glasses.

The term "thin film metallic glass" refers to metallic materials that are amorphous and lack the crystalline structure typically found in conventional metals. The amorphous or non-crystalline structure of thin film metallic glasses provides distinct properties, such as high strength, hardness, and corrosion resistance.

The "atomic percent" refers to the proportion of the total number of atoms of a specific element in an alloy or compound, expressed as a percentage of the total number of atoms in the mixture. In other words, it describes the fraction of atoms of a particular element relative to the total number of atoms of all elements present in the material.

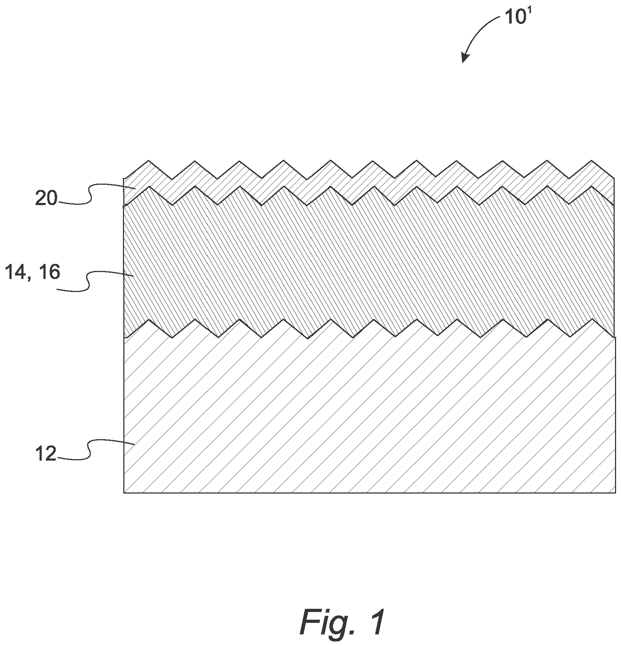

Referring to Figure 1, a schematic cross-section of a coated article is provided. Coated article 10¹ includes a substrate 12. A doped thin film metallic glass (TFMG) 16 and/or undoped TFMG layer 14 is disposed over and optionally contacts substrate 12. Top layer 20 is disposed over and optionally contacts undoped TFMG layer 14. Advantageously, the top layer includes a metal-containing layer and/or a carbon-containing layer.

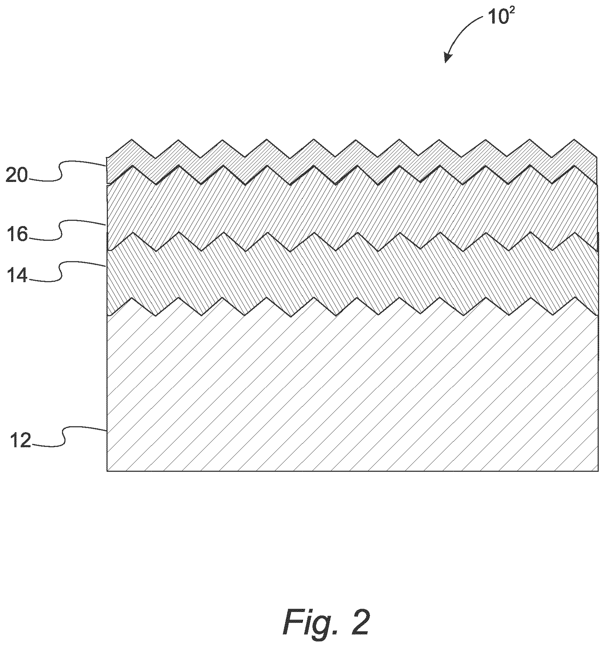

In a variation as depicted in Figure 2, a doped TFMG layer is interposed between the undoped TFMG layer and the top layer. Coated article 10² includes a substrate 12. Undoped TFMG layer 14 is disposed over and optionally contacts substrate 12. Doped TFMG layer 16 is disposed over and optionally contacts undoped TFMG layer 14. Top layer 20 is disposed over and optionally contacts doped TFMG layer 16. Doped TFMG layer 16 is a TFMG layer that includes a dopant to modify such properties as stability, hardness, corrosion resistance, and the like. In general, TFMG layers include a TFMG composition as set forth herein and a dopant. In this context, a dopant is a foreign element that is intentionally added in small quantities to a TFMG to modify its properties. In a refinement, the dopant can be present in an amount from 0.01 weight percent to 5 weight percent of the total weight of the doped TFMG layer. Alternatively, the dopant can be present in an amount from 0.5 atom percent to 20 atom percent. In some refinements, the dopant can be present in an amount of at least 0.2 atom percent, 0.5 atom percent, 1 atom percent, 2 atom percent, 3 atom percent, or 5 atom percent, and at most 20 atom percent, 18 atom percent, 15 atom percent, 10 atom percent, 8 atom percent, 5 atom percent, or 3 atom percent.

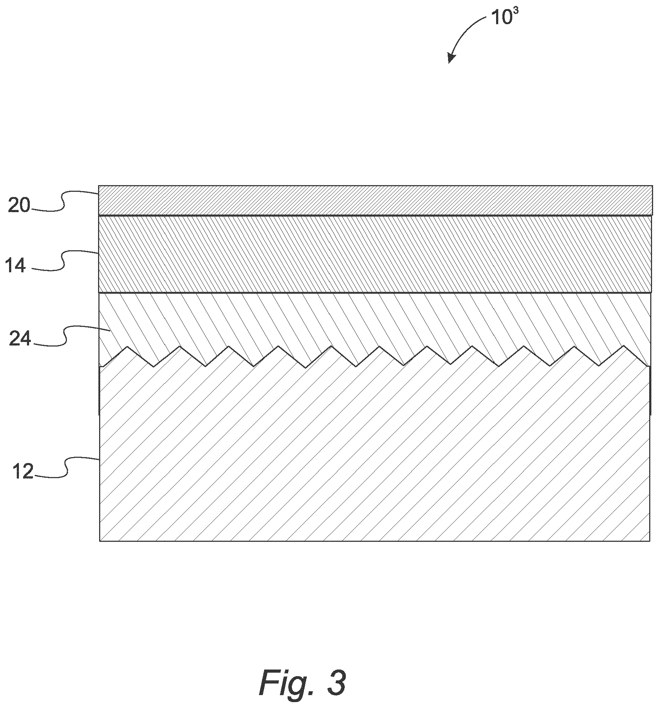

In a variation as depicted in Figure 3, a leveling primer layer is interposed between the substrate and the undoped TFMG layer. Coated article 10³ includes a substrate 12. Leveling primer layer 24 is disposed over and optionally contacts substrate 12. Undoped TFMG layer 14 is disposed over and optionally contacts leveling primer layer 24. Top layer 20 is disposed over and optionally contacts undoped TFMG layer 14. As set forth above, the top layer includes a metal-containing layer or carbon-containing layer.

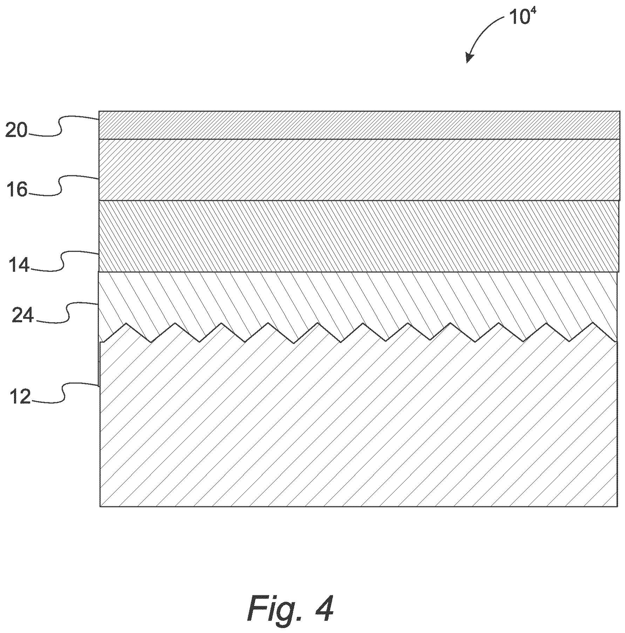

In a variation as depicted in Figure 4, a leveling primer layer interposed between the substrate and the undoped TFMG layer and a doped TFMG layer interposed between the undoped TFMG layer and the metal layer. Coated article 10⁴ includes a substrate 12. Leveling primer layer 24 is disposed over, and optionally contacts substrate 12. Undoped TFMG layer 14 is disposed over and optionally contacts leveling primer layer 24. Doped TFMG layer 16 is disposed over and optionally contacts undoped TFMG layer 14. Top layer 20 is disposed over, and optionally contacts doped TFMG layer 16. As set forth above, the top layer includes a metal-containing layer or carbon-containing layer.

Referring to Figures 1, 2, 3, and 4, each of the coated articles includes a top layer that is a metal-containing layer or carbon-containing layer. In a refinement, the top layer is a metal layer, a metal compound layer, or a metal alloy layer. The metal layer or metal alloy layer or metal compound layer can be formed by physical vapor deposition techniques such as cathodic arc deposition, magnetron sputtering, evaporation, or the like. In a further refinement, the top layer includes metallic chromium, metallic nickel, or alloys thereof. In another refinement, the top layer includes a component selected from the group consisting of ZrN, ZrCN, ZrO2, ZrOC, diamond-like carbon (DLC), and combinations thereof. Typically, the top layer has a thickness from about 0.2 microns to 1 micron. In some refinement, the top layer has a thickness of at least 0.1 microns, 0.2 microns, 0.3 microns, 0.4 microns, or 0.5 microns and a thickness of at most 2 microns, 1.5 microns, 1 micron, 0.7 microns, or 0.6 microns.

Referring to Figures 1, 2, 3, and 4, each of the coated articles includes a undoped TFMG layer and/or an optional doped TFMG layer. Typically, the undoped TFMG layer 14 and the optionally doped TFMG layer 16 each independently have a thickness from about 0.5 to 10 microns. In some refinement, the undoped TFMG layer 14 and the optionally doped TFMG layer 16 each independently have a thickness of at least 0.3 microns, 0.5 microns, 1 micron, 1.5 microns, 2 microns, 3 microns, 4 microns, or 5 microns and at most 10 microns, 9 microns, 8 microns, 7 microns, 6 microns, or 5 microns.

In another aspect, the undoped TFMG layer 14 and the optionally doped TFMG layer 16 independently include a base element such as zirconium, copper, nickel, titanium, molybdenum, or combinations thereof. In this context, a base element is the element with the greatest atomic percent. In a refinement, the undoped TFMG layer 14 and the optionally doped TFMG layer 16 independently include a base element and an alloying element selected from the group consisting of copper, aluminum, titanium, silicon, nickel, boron, tungsten, chromium, and combinations thereof. In a refinement, the undoped TFMG layer 14 and the optionally doped TFMG layer 16 are independently based on zirconium (e.g., include zirconium as the primary element). In general, zirconium-based TMFGs have about 50 to 65 atom% zirconium. In a further refinement, the undoped TFMG layer 14 and the optionally doped TFMG layer 16 independently include a zirconium-copper alloy typically having 50 to 65 atom% zirconium and 15 to 30 percent copper with any balance being additional alloying elements or dopants. Examples of zirconium-based systems include Zr-Cu-Al-N compositions such as Zr₆₀Cu₂₄Al₁₁Ni₅, Zr-Ti-B-Si compositions, and Zr-Ti-Ni compositions such as Zr₄₆Ti₂₆Ni₂₈. It should be appreciated that any subscript for a chemical formula can be practiced within 20 %, 10%, 5 %, or 1 % of the indicated value. In a refinement, Zr-Cu-Al-N compositions include 50-65 atom% zirconium, 15-30 atom% copper, 5-15 atom% aluminum, and nickel in an amount of 2-10 atom% nickel. In refinement, Zr-Ti-B-Si compositions include 50-60 atom% zirconium, 10-30 atom% titanium, 1-10 atom% boron, and 5-15 atom% silicon. In a refinement, Zr-Ti-Ni compositions include 40-60 atom% zirconium, 10-30 atom% titanium, and 5-20 atom% nickel. In another refinement, the undoped TFMG layer 14 and the optionally doped TFMG layer 16 are independently a Zr-B system. In a refinement, the Zr-B system includes 60 to 90 atom% zirconium and 5 to 40 percent boron, with any balance being additional alloying elements or dopants. In a refinement, the Zr-B system is combined with titanium and silicon to form a Zr-Ti-B-Si composition. In a further refinement, Zr-Ti-B-Si composition includes 50-65 atom% zirconium, 5-15 atom% boron, 10-30 atom% titanium, and 5-15 atom% silicon.

In another aspect, the undoped TFMG layer 14 and the optionally doped TFMG layer 16 are independently a cobalt system such as a Mo-Co-B composition. In a refinement, the Mo-Co-B composition includes 40-60 atom% molybdenum, 20-40 atom% cobalt, and 5-20 atom% boron with any balance being additional alloying elements or dopants.

Referring to Figures 1, 2, 3, and 4, examples of dopants include but are not limited to nitrogen (N), oxygen (O), carbon (C), silicon (Si), and the like. Nitrogen is often introduced as a dopant to improve mechanical properties like hardness and wear resistance. The strong electronegativity of nitrogen leads to a denser and more tightly packed structure in the TFMG, which reduces atomic spacing and enhances hardness and thermal stability. Nitrogen also contributes to improved corrosion resistance in environments with high humidity or salinity. In a refinement, nitrogen is present in an amount of 1 to 10 atom%. Oxygen is commonly used as a dopant to enhance the corrosion resistance of TFMGs. By forming oxide layers or introducing oxygen into the amorphous matrix, TFMGs become more resistant to oxidation and environmental degradation. Oxygen also helps in passivating the surface, reducing reactivity in aggressive environments. In a refinement, oxygen is present in an amount of 1 to 5 atom%. Carbon doping can significantly improve hardness and mechanical strength. This is particularly common in applications where wear resistance is critical, such as cutting tools or high-friction environments. Carbon can also contribute to improved tribological properties by reducing friction. In a refinement, silicon is present in an amount of 0.5 to 5 atom%. Silicon is often added to improve the thermal and oxidation resistance of TFMGs. It forms a protective oxide layer on the surface, making the material more stable at elevated temperatures and in corrosive environments. Si-doped TFMGs are often used in high-temperature applications or where long-term stability in harsh environments is required. In a refinement, silicon is present in an amount of 5 to 20 atom%. In another aspect, the top layer 14 includes a metal-containing layer or carbon-containing layer as set forth below in more detail.

Additional details regarding the TFMGs utilized in the multilayer coatings can be found in the reference: Pakman Yiu, Wahyu Diyatmika, Niklas Bönninghoff, et al., "Thin film metallic glasses: Properties, applications, and future," Journal of Applied Physics, vol. 127, no. 030901, 2020, DOI: 10.1063/1.5122884. TFMGs provide a unique combination of smooth surface, superior hardness, corrosion resistance, and low surface energy, making them ideal for multilayer coatings requiring durability, aesthetics, and protection against environmental degradation.

The composition of TFMGs can be customized to meet specific application needs. The flexibility in tuning the composition of TFMGs allows for property optimization to suit a variety of applications, such as enhancing thermal stability for high-temperature environments or improving biocompatibility for use in medical devices. In addition to their functional advantages, TFMGs present an environmentally friendly alternative to conventional electroplating techniques, which often involve hazardous chemicals and pose environmental risks. As described below in more detail, TFMGs include common elements such as zirconium (Zr), titanium (Ti), copper (Cu), nickel (Ni), and aluminum (Al). For example, Zr-based TFMGs demonstrate exceptional resistance to wear and corrosion, making them suitable for environments exposed to moisture or chemicals. Their smooth surface also reduces friction, which is beneficial for applications where low surface energy is important to prevent fouling.

TFMGs also exhibit mechanical properties like high hardness, often exceeding 9 GPa, which allows them to act as effective protective layers in multilayer coatings. These properties remain stable even after thermal treatment, making TFMGs suitable for applications involving heat-intensive processes.

Furthermore, TFMGs form highly adherent layers on various substrates, improving the durability and longevity of the overall coating. Their amorphous nature acts as a barrier, preventing interfacial degradation by inhibiting the diffusion of elements between layers.

Overall, the versatility of TFMGs makes them suitable for a wide range of applications, from industrial protective coatings to biomedical devices, due to their tunable mechanical, thermal, and surface properties.

Referring to Figures 1, 2, 3, and 4, each of the coated articles includes a substrate. Although the coated article is not limited by the type of substrate, examples of substrates are composed of brass, stainless steel, aluminum, zinc, or a polymer (e.g., ABS).

Referring to Figures 3 and 4, each of the coated articles includes a leveling primer layer 24. The leveling primer layer 24 can be a copper metal layer, a siloxane coating, or a polymer coating. In a refinement, the leveling primer layer 24 has a thickness from about 1 to 25 microns. In some refinements, the leveling primer layer has a thickness of at least 0.2 microns, 0.5 microns, 1 microns, 2 microns, 3 microns, or 5 microns, and at least 25 microns, 20 microns, 15 microns, 10 microns, 8 microns, or 7 microns

In another aspect, the top 20 is PVD/PE-CVD coatings for a range of different colors and added durability. In some refinements, the metal-containing layer includes metallic chromium, metallic nickel, or alloys thereof. In a variation, the metal-containing layer includes a component selected from the group consisting of ZrN, ZrCN, ZrO₂, ZrOC, diamond-like carbon (DLC), and combinations thereof.

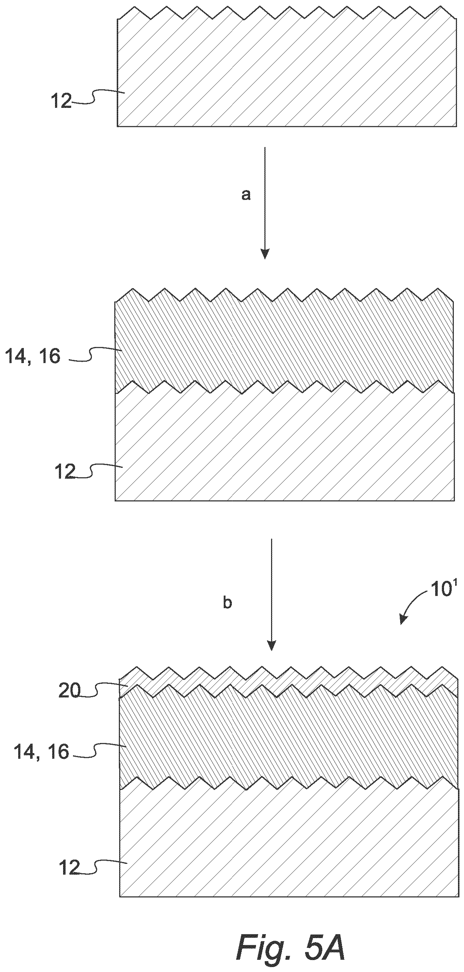

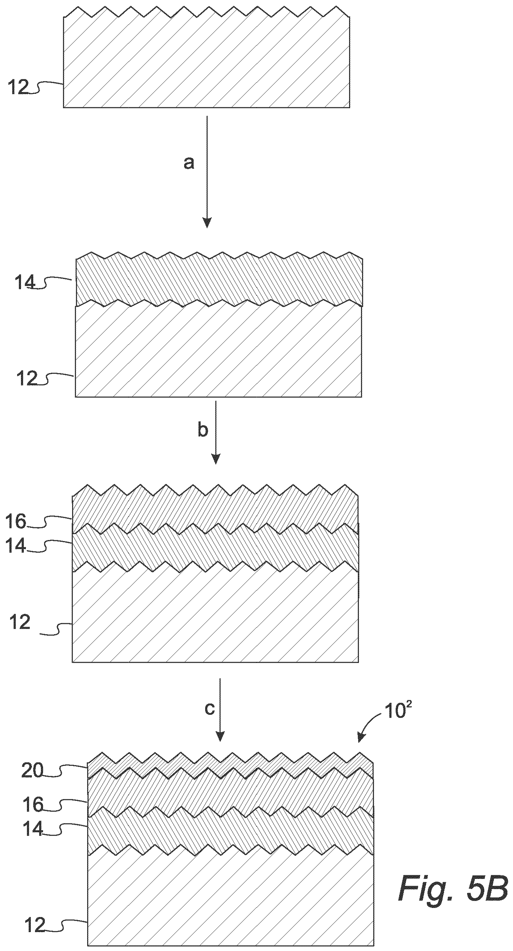

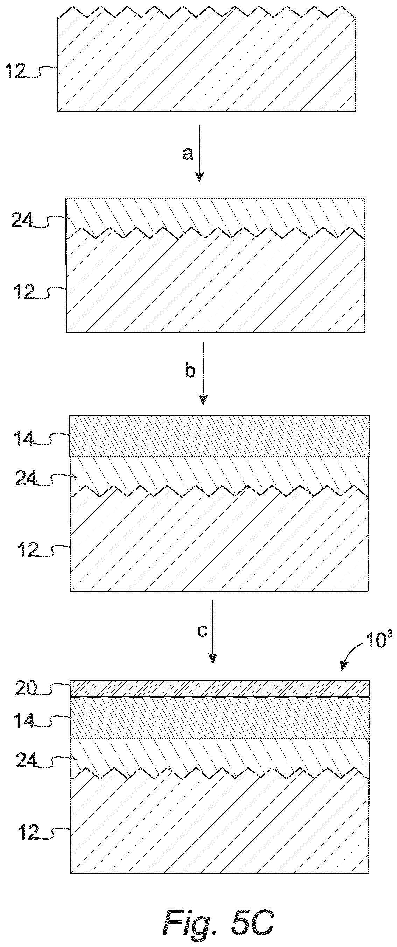

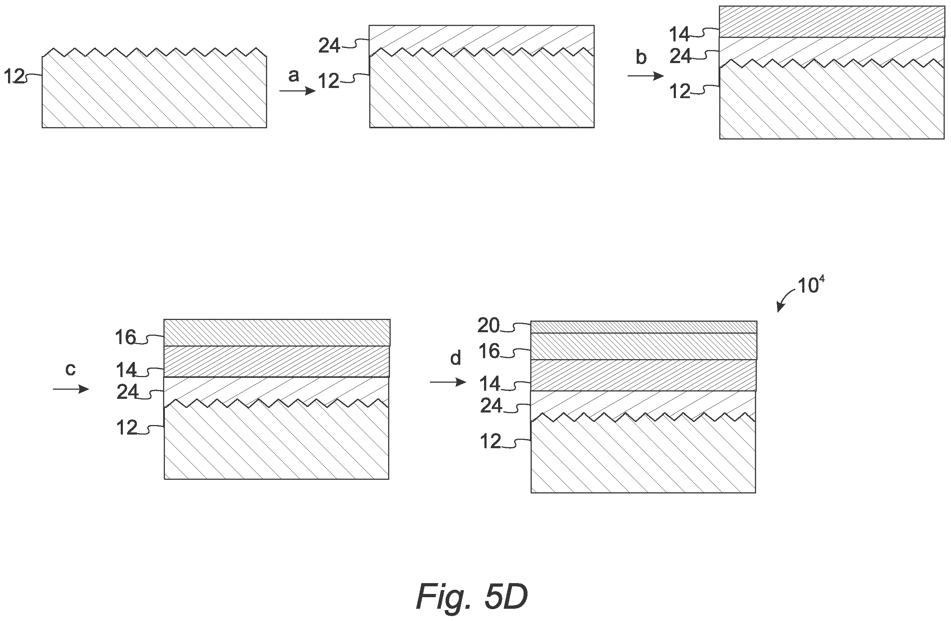

In another aspect, a method for making a coated article of Figures 1, 2, 3, and 4 is provided. Figures 5A, 5B, 5C, and 5d, provide flowcharts depicting methods of making the coated article are provided. Referring to Figure 5A, a doped thin film metallic glass layer 16 or undoped thin film metallic glass layer 14 is deposited over and optionally contacting a substrate 12 in step a). Typically, doped thin film metallic glass layer 16 and/or undoped thin film metallic glass layer 14 are deposited by physical vapor deposition. In step b), a top layer 20 is deposited by physical vapor deposition over and optionally contacting the doped thin film metallic glass layer 16 or undoped thin film metallic glass layer 14 to form coated article 10¹. Typically, doped thin film metallic glass layer 16 and/or undoped thin film metallic glass layer 14 are deposited by physical vapor deposition.

Referring to Figure 5B, an undoped thin film metallic glass layer 14 is deposited over and optionally contacting a substrate 12 in step a). In step b), a doped thin film metallic glass layer 16 is deposited over and optionally contacting undoped thin film metallic glass layer 14. In step c), a top layer 20 is deposited by physical vapor deposition over and optionally contacting the doped thin film metallic glass layer 16 to form coated article 10². Typically, doped thin film metallic glass layer 16 and/or undoped thin film metallic glass layer 14 are deposited by physical vapor deposition.

Referring to Figure 5C, leveling primer layer 24 is deposited over and optionally contacting a substrate 12 in step a). In step b), undoped thin film metallic glass layer 14 is deposited over and optionally contacting leveling primer layer 24. In step c), a top layer 20 is deposited by physical vapor deposition over and optionally contacting undoped thin film metallic glass layer 14. Typically, undoped thin film metallic glass layer 14 is deposited by physical vapor deposition.

Referring to Figure 5D, leveling primer layer 24 is deposited over and optionally contacting a substrate 12 in step a). In step b), undoped thin film metallic glass layer 14 is deposited over and optionally contacting leveling primer layer 24. In step c), doped thin film metallic glass layer 16 is deposited over and optionally contacting undoped thin film metallic glass layer 14. In step d), a top layer 20 is deposited by physical vapor deposition over and optionally contacting doped thin film metallic glass layer 16. Typically, doped thin film metallic glass layer 16 and/or undoped thin film metallic glass layer 14 are deposited by physical vapor deposition.

Referring to Figures 5A, 5B, 5C, and 5D, examples of physical vapor deposition (PVD) techniques include magnetron sputtering or cathodic arc deposition, which enable the creation of smooth, defect-free surfaces that contribute to their superior hardness and corrosion resistance. As set forth above, the top layer includes a metal-containing layer and/or a carbon-containing layer. Examples of physical vapor deposition methods include magnetron sputtering, high-power impulse magnetron sputtering, cathodic arc deposition, or a combination thereof.

In another aspect, the undoped TFMG and the doped TFMG layers can be deposited in a standard PVD system by conventional magnetron sputtering, HiPIMS, cathodic arc deposition, or combinations. The target materials used in the PVD systems can be alloys fabricated to specific desired alloy compositions, or the films could be formed by co-deposition (e.g., co-sputtering) from individual pure or alloy metal targets (e.g., Zr-Cu formed by magnetron sputtering Cu and cathodic arc of Zr running simultaneously). Additionally, the doped TFMG layer can be formed by introducing reactive gases (N₂, O₂, CH₄) during the deposition process at controlled rates to produce the desired compositions. Additional details about the properties of TFMG layers and method for fabrication are set forth in Pakman Yiu, Wahyu Diyatmika, Niklas Bönninghoff, et al., "Thin film metallic glasses: Properties, applications, and future," Journal of Applied Physics, vol. 127, no. 030901, 2020. DOI: 10.1063/1.5122884.

The coatings set forth above can be applied directly to substrates, including but not limited to brass, stainless steel, aluminum, zinc, and ABS. Substrates may be treated with coatings such as electroless Cu plating, siloxane coatings, and polymer coatings for surface leveling and adhesion promotion.

In another aspect, coated articles of Figures 1, 2, 3, and 4 can be applied in a number of applications. The coated articles can used for medical instruments, including surgical blades and needles. The coating improves the sharpness, durability, and nonstick properties of instruments, reducing the force required for insertion and minimizing tissue damage. The coatings can be used as diffusion barriers in electronics since TFMGs are effective as diffusion barriers in semiconductor and electronic devices. The coatings can be used for corrosion resistance because of the amorphous nature of TFMGs. The coated articles can be used in consumer products because of their smooth surface, hardness, and nonstick properties. In other applications, the substrate can be a flexible polymer substrate. Coated articles with flexible substrates can be used for flexible electronics.

While exemplary embodiments are described above, it is not intended that these embodiments describe all possible forms of the invention. Rather, the words used in the specification are words of description rather than limitation, and it is understood that various changes may be made without departing from the scope of the invention. Additionally, the features of various implementing embodiments may be combined to form further embodiments of the invention.

## Claims

1. A coated article comprising:
a substrate (12);
a doped thin film metallic glass (TFMG) layer (16) and/or undoped thin film metallic glass (TFMG) layer (14) disposed over the substrate (12); and
a top layer (20) disposed over the doped TFMG layer (16) and/or the undoped TFMG layer (14), the top layer (20) including a metal-containing layer and/or a carbon-containing layer.

2. The coated article of claim 1, wherein the top layer (20) is a metal layer or a metal alloy layer or a metal compound layer, and optionally wherein the top layer (20) includes metallic chromium, metallic nickel, or alloys thereof.

3. The coated article of claim 1 or claim 2, wherein the top layer (20) includes a component selected from the group consisting of ZrN, ZrCN, ZrO2, ZrOC, diamond-like carbon (DLC), and combinations thereof, and optionally wherein the top layer (20) has a thickness from about 0.2 microns to 1 micron.

4. The coated article of any of claims 1 to 3, wherein the TFMG layer (16) has a thickness from about 0.5 to 10 microns.

5. The coated article of any of claims 1 to 4, wherein the TFMG layer (16) comprises a base element selected from the group consisting of zirconium, copper, nickel, titanium, molybdenum, and combinations thereof, and optionally wherein the TFMG layer (16) further comprises an alloying element selected from the group consisting of copper, aluminum, titanium, silicon, nickel, boron, tungsten, chromium, and combinations thereof.

6. The coated article of any of claims 1 to 5, wherein the TFMG layer (16) includes zirconium, and optionally wherein the TFMG layer (16) is a zirconium-copper alloy having 50 to 65 atom% zirconium and 15 to 30 atom% copper, with any balance being additional alloying elements or dopants.

7. The coated article of any of claims 1 to 6, wherein the doped TFMG layer (16) is interposed between the undoped TFMG layer (14) and the top layer (20), and optionally wherein the doped TFMG layer (16) has a thickness from about 0.5 to 10 microns.

8. The coated article of claim 7, wherein the doped TFMG layer (16) includes a TFMG composition and a dopant, and optionally wherein the dopant is selected from the group consisting of N, C, O, and combinations thereof.

9. The coated article of any of claims 1 to 8 further comprising a leveling primer layer (24) interposed between the substrate (12) and the undoped TFMG layer (14).

10. The coated article of claim 9, wherein the leveling primer layer (24) is a copper metal layer, a siloxane coating, or a polymer coating, and optionally wherein the leveling primer layer (24) has a thickness from about 1 to 25 microns.

11. The coated article of claim 9 or claim 10, further comprising a doped TFMG layer (16) interposed between the undoped TFMG layer (14) and the top layer (20).

12. The coated article of any of claims 1 to 11, wherein the substrate (12) is composed of brass, stainless steel, aluminum, zinc, or a polymer.

13. A method for making a coated article comprising:
depositing a doped thin film metallic glass (TFMG) layer (16) and/or undoped thin film metallic glass (TFMG) layer (14) over a substrate (12); and
depositing a top layer (20) by physical vapor deposition over the doped TFMG layer (16) and/or the undoped TFMG layer (14), the top layer (20) including a metal-containing layer or carbon-containing layer.

14. The method of claim 13, wherein the doped TFMG layer (16) and/or the undoped TFMG layer (14) is formed by magnetron sputtering, high-power impulse magnetron sputtering, cathodic arc deposition, or a combination thereof.

15. The method of claim 13 or claim 14, wherein the top layer is a metal layer or a metal alloy layer or a metal compound layer, and optionally wherein the top layer is formed by cathodic arc deposition.
